# EUROPÄISCHE PATENTSCHRIFT

(11) **EP 1 531 826 B1**
(45) Veröffentlichungstag und Bekanntmachung des Hinweises auf die Patenterteilung: **11.05.2011**
(21) Anmeldenummer: 03792402.4
(22) Anmeldetag: 21.08.2003
(51) Int. Cl.: A61K 31/55, A61P 31/00, A61K 31/135

(54) **ANTIDEPRESSIVA ZUR PROPHYLAXE UND THERAPIE VON ZYSTISCHER FIBROSE**
ANTIDEPRESSANTS FOR THE PROPHYLAXIS AND TREATMENT OF CYSTIC FIBROSIS
ANTIDEPRESSEURS POUR LA PROPHYLAXE ET THERAPIE DE LA MUCOVISCIDOSE

(30) Priorität: 23.08.2002 DE 10239531
(43) Veröffentlichungstag der Anmeldung: 25.05.2005
(73) Patentinhaber: Gulbins, Erich, Prof. Dr., 45122 Essen (DE); Adams, Claus, Prof. Dr., 26409 Wittmund (DE)
(72) Erfinder: Gulbins, Erich, 45122 Essen (DE)
(74) Vertreter: Patentanwälte Ruff, Wilhelm, Beier, Dauster & Partner
(86) Internationale Anmeldenummer: PCT/EP2003/009254
(87) Internationale Veröffentlichungsnummer: WO 2004/017949

(56) Entgegenhaltungen:
- WO-A-00/33885
- WO-A-00/56890
- WO-A-90/14089
- WO-A-92/18162
- WO-A-99/41373
- WO-A-03/061767
- US-A1- 2002 177 126
- PRINCE L S ET AL: "KGF alters gene expression in human airway epithelia: potential regulation of the inflammatory response." PHYSIOLOGICAL GENOMICS 17 JUL 2001, Bd. 6, Nr. 2, 17. Juli 2001 (2001-07-17), Seiten 81-89, XP008077228 ISSN: 1531-2267
- SHUTE J ET AL: "Growth factors in cystic fibrosis - When more is not enough" PAEDIATRIC RESPIRATORY REVIEWS 2003 UNITED KINGDOM, Bd. 4, Nr. 2, 2003, Seiten 120-127, XP008077229 ISSN: 1526-0550
- CLAUS R ET AL: "Modulation of the ceramide level, a novel therapeutic concept?" CURRENT DRUG TARGETS. NETHERLANDS SEP 2000, Bd. 1, Nr. 2, September 2000 (2000-09), Seiten 185-205, XP008023836 ISSN: 1389-4501
- MUNOZ-BELLIDO J L ET AL: "ANTIMICROBIAL ACTIVITY OF PSYCHOTROPIC DRUGS SELECTIVE SEROTONIN REUPTAKE INHIBITORS" INTERNATIONAL JOURNAL OF ANTIMICROBIAL AGENTS, AMSTERDAM, NL, Bd. 14, Nr. 3, April 2000 (2000-04), Seiten 177-180, XP001022171 ISSN: 0924-8579
- KRISTIANSEN J E ET AL: "INHIBITION OF HIV REPLICATION BY NEUROLEPTIC AGENTS AND THEIR POTENTIAL USE IN HIV INFECTED PATIENTS WITH AIDS RELATED DEMENTIA" INTERNATIONAL JOURNAL OF ANTIMICROBIAL AGENTS, AMSTERDAM, NL, Bd. 14, Nr. 3, 2000, Seiten 209-213, XP001056998 ISSN: 0924-8579
- DUTTA, PURABI ET AL: "Antimalarial properties of imipramine and amitriptyline" JOURNAL OF PROTOZOOLOGY (1990), 37(1), 54-8, XP008023929
- LAUER, SABINE A. ET AL: "Sphingolipid synthesis as a target for chemotherapy against malaria parasites" PROCEEDINGS OF THE NATIONAL ACADEMY OF SCIENCES OF THE UNITED STATES OF AMERICA (1995), 92(20), 9181-5, XP001172814
- HAUCK C R ET AL: "Acid sphingomyelinase is involved in CEACAM receptor-mediated phagocytosis of Neisseria gonorrhoeae" FEBS LETTERS, Bd. 478, Nr. 3, 4. August 2000 (2000-08-04), Seiten 260-266, XP004337445 ISSN: 0014-5793
- BAUMANN, NICOLE ET AL: "Effect of tricyclic antidepressants on lysosomal sphingomyelinase activit" NATO ASI SERIES, SERIES A: LIFE SCIENCES (1988), 150(LIPID STORAGE DISORD.), 627-34, XP008023933
- ALBOUZ, S. ET AL: "Effect of tricyclic antidepressants on sphingomyelinase and other sphingolipid hydrolases in C6 cultured glioma cells" NEUROSCIENCE LETTERS (1983), 36(3), 311-15, XP008023932

## Beschreibung

Die vorliegende Erfindung betrifft die Verwendung von Wirkstoffen, die zur Prophylaxe und/oder Therapie von zystischer Fibrose geeignet sind.

Da Infektionskrankheiten nach wie vor weltweit ein sehr großes medizinisches Problem darstellen, sind Studien zur Behandlung und zur Prophylaxe derartiger Erkrankungen schon lange Gegenstand intensiver Forschung. Beispielsweise werden ständig immense Kosten aufgewendet, um neue Antibiotika zu entwickeln. Diese Antibiotika sind notwendig, um Bakterien, Pilze, Protozoen oder Parasiten als Erreger von Infektionskrankheiten bekämpfen zu können. Insbesondere muß hierbei den sich laufend neu entwickelnden Resistenzen der Erreger Rechnung getragen werden, was zunehmend problematischer wird.

Eine besondere Schwierigkeit stellt die Behandlung von Infektionskrankheiten dar, die durch Viren oder Prionen verursacht sind. Aufgrund eines fehlenden eigenen Stoffwechsels sind diese Erreger nicht mit Antibiotika angreifbar, so daß im allgemeinen nur eine Behandlung der Symptome möglich ist.

Um die Prophylaxe und Therapie von Infektionskrankheiten zu verbessern und insbesondere den erwähnten Problemen Rechnung zu tragen, stellt sich die Erfindung die Aufgabe, Wirkstoffe bereitzustellen, die in besonderer Weise zur Prophylaxe und Therapie von zystischer Fibrose geeignet sind.

Die Aufgabe wird gelöst durch eine Verwendung von Hemmstoffen, wie sie in den Ansprüchen 1 und 2 beschrieben ist.

Erfindungsgemäß werden zur Prophylaxe und/oder Therapie von zystischer Fibrose zysticher Fibrose Hemmstoffe der sauren Sphingomyelinase und/oder Hemmstoffe von Produkten, die durch die von der sauren Sphingomyelinase katalysierte Reaktion entstehen gemäss den Ansprüchen, verwendet. Zu diesen Produkten zählt insbesondere Ceramid, welches durch die Spaltung von Sphingolipiden entsteht. Versuche, die zu dieser neuen Verwendung von Hemmstoffen dieser Art geführt haben, zeigten, daß hierdurch die Infektion eukaryotischer Zellen mit verschiedenen pathogenen Organismen, beispielsweise Bakterien, Viren, Pilze und Parasiten, effektiv verhindert werden kann. Neben der Prophylaxe und/oder Therapie von Infektionskrankheiten können die genannten Hemmstoffe auch mit Vorteil zur Prophylaxe und/oder Therapie von Krankheiten eingesetzt werden, deren klinischer Verlauf durch Infektionen zumindest mitbestimmt wird, nämlich zystischer Fibrose.

Diese überraschenden Ergebnisse beruhen darauf, daß ceramidreiche Membranplattformen in der Zellmembran von eukaryotischen Zellen für eine Infektion eukaryotischer Zellen mit pathogenen Erregern notwendig sind. Diese größeren Plattformen in der Zellmembran der eukaryotischen Zellen werden durch Fusion von sehr kleinen distinkten Domänen in der Zellmembran, sogenannten Rafts, gebildet. Diese Rafts bestehen aus Cholesterol und Sphingolipiden, insbesondere Sphingomyelin, die sehr fest miteinander assoziieren, wodurch sie sich von den Phospholipiden der Zellmembran trennen und diese kleinen distinkten Domänen bilden. Das in Rafts am häufigsten vorkommende Sphingolipid ist Sphingomyelin, das aus dem sehr hydrophoben Ceramidrest und der hydrophilen Phosphorylcholin-Kopfgruppe besteht. Ceramid ist ein Amidester aus der Sphingoidbase D-erythro-Sphingosin und einer Fettsäure, normalerweise mit einer Kettenlänge von C₁₆ bis C₂₆. Wasserstoffbrücken-Bindungen und hydrophobe van der Waal-Interaktionen zwischen dem Cholesterol-Ringsystem und Sphingolipiden bzw. zwischen den Kopfgruppen der Sphingolipide führen zu einer lateralen Assoziation der Sphingolipide und des Cholesterols in der Zellmembran und einer spontanen Separation von den übrigen Phospholipiden (Brown D.A., London E. (1998). Functions of lipid rafts in biological membranes. Annu. Rev. Cell. Dev. Biol. 14: 111-367; Harder T., Simons K. (1997). Caveolae, DIGs, and the dynamics of sphingolipidcholesterol microdomains. Curr. Opin. Cell. Biol. 9: 534-542). Dadurch entstehen die als Rafts bezeichneten sehr kleinen, distinkten Sphingolipid- und Cholesterol-reichen Membrandomänen. Extrahiert man aus Rafts das Cholesterol, das wahrscheinlich als Spacer zwischen den Sphingolipiden mit ihren relativ grossen Kopfgruppen fungiert, so wird die Struktur und Funktion von Rafts zerstört

Erfindungsgemäß wurde ein Mechanismus identifiziert, der die Bildung von Membranplattformen aus Rafts, das Clustern bzw. Aggregieren von Rezeptoren in diesen Membranplattformen und die Infektion von Zellen mit pathogenen Bakterien und Viren vermittelt. Beispielsweise nach Stimulation über CD95 oder den CD40 Rezeptor, Infektion mit *Pseudomonas aeruginosa, Staphylococcus aureus, Neisseriae gonorhoeae* oder auch Infektion menschlicher Zellen mit Rhinoviren kommt es in Rafts zu einer Freisetzung von Ceramid aus Sphingomyelin. Die Bildung von Ceramid in Rafts führt auf Grund der biophysikalischen Eigenschaften von Ceramid zu einer Fusion kleiner Rafts zu grossen, ceramidreichen Plattformen in der Zellmembran.

Ergebnisse, die zur Erfindung führten, zeigten, daß diese kleinen Rafts durch das Enzym saure Sphingomyelinase und durch Ceramid, das durch die von diesem Enzym katalysierte Reaktion freigesetzt wird, zu den erwähnten größeren Plattformen fusioniert werden.

Eine physiologische Bedeutung dieser Plattformen wurde bereits kürzlich von Grassmé et al. beschrieben (J. Biol. Chem. 276, 20589-20596 (2001); J. Immunol. 168, 298-307 (2002)). Die Autoren konnten zeigen, daß die ceramidreichen Plattformen der Vermittlung von Signalen aus dem Extrazellulärraum in das Zellinnere dienen. Hierbei wird zunächst durch Aktivierung verschiedener Rezeptoren, z. B. CD95 und CD40, eine Translokation des Enzyms saure Sphingomyelinase hin zur Außenseite der Membran induziert. Hier setzt die saure Sphingomyelinase aus Sphingomyelin Ceramid frei, das spontan in Rafts aggregiert. Dadurch kommt es zur Transformation von Rafts in sehr hydrophobe Membranbereiche. Diese Ceramidaggregate zeigen weiterhin die Tendenz, spontan zu größeren Membranplattformen zu fusionieren. In diesen ceramidreichen Membranplattformen aggregieren aktivierte Rezeptoren wie CD95 und CD40, was für die Vermittlung eines Signals über diese Rezeptoren in die Zelle notwendig ist.

Interessanterweise konnte nun im Rahmen der Erfindung gezeigt werden, daß diese Plattformen zusätzlich ein Eindringen von pathogenen Organismen über diese entsprechenden Membranabschnitte ermöglichen. An der Bildung dieser "Eintrittstüren" für pathogene Organismen in die eukaryotische Zelle ist das Enzym saure Sphingomyelinase entscheidend beteiligt, da Ceramide als Produkte der von diesem Enzym katalysierten Reaktion die Membranplattformen bilden. Ausgelöst wird dieser Vorgang dadurch, daß durch die Erreger über einen bisher noch nicht bekannten Mechanismus die saure Sphingomyelinase dazu veranlaßt wird, in intrazellulären Vesikeln an die Zelloberfläche bzw. in die äußere Seite der Membran transportiert zu werden. Dort wird durch das Enzym der Abbau von Sphingomyelin zu Ceramid in der Membran bewirkt. Diese Bedeutung ceramidreicher Membranplattformen für Infektionen mit pathogenen Bakterien und Viren konnte von den Erfindern insbesondere am Beispiel der Infektion mammalischer Zellen mit *Pseudomonas aeruginosa* gezeigt werden. Die Infektion mammalischer Zellen mit *P. aeruginosa* aktiviert die saure Sphingomyelinase und induziert damit die Freisetzung von Ceramid.

Eine weitere Aufgabe des Enzyms saure Sphingomyelinase wurde bereits im Zusammenhang mit der Invasion von Gonokokken in eukaryotischen Zellen von Grassmé et al. beschrieben (Cell, Vol. 91, 605-615, 1997). Die Autoren konnten zeigen, daß die saure Sphingomyelinase an einer Signaltransduktionskette in der Wirtszelle beteiligt ist, welche die Invasion eines bestimmten Gonokokken-Stammes vermittelt. Entscheidend sind hierbei bestimmte Oberflächenproteine auf dem bakteriellen Erreger, die sogenannten Opa-Proteine. Diese binden an einen spezifischen Rezeptor der eukaryotischen Wirtszelle, so daß eine Phospholipase C aktiviert und Diacylglycerol gebildet wird. Dieses Diacylglycerol aktiviert wiederum die saure Sphingomyelinase, so daß durch die von diesem Enzym katalysierte Reaktion Ceramid gebildet wird. Diese Signalkaskade konnte jedoch nur durch ganz bestimmte Bakterien bzw. Stämme induziert werden. Insbesondere war die Induzierbarkeit dieser Signalkaskade abhängig von den Opa-Proteinen auf dem Bakterium. Es handelt sich hierbei also um einen völlig anderen, sehr speziellen Mechanismus im Vergleich mit dem im Zusammenhang mit der Erfindung entdeckten generellen Invasions- bzw. lnfektionsmechanismus über die besagten Membranplattformen.

Durch die erfindungsgemäße Verhinderung der Ausbildung der Membranplattformen und/oder der Zerstörung bereits gebildeter Membranplattformen wird ein Eindringen der Erreger in die Wirtszelle verhindert. Dies wird im Rahmen der Erfindung vorzugsweise durch die Verwendung von Hemmstoffen gemäss der Ansprüchen der sauren Sphingomyelinase und/oder von Hemmstoffen gemäss der Ansprüchen der Produkte der von diesem Enzym katalysierten Reaktion erreicht. Dies hat gegenüber herkömmlichen Behandlungsmethoden von Infektionskrankheiten den Vorteil, daß sich das Target für den Wirkstoff auf der Wirtszelle, also der zu infizierenden eukaryotischen Zelle, befindet. Es wird hiermit offenbart ein genereller Infektionshemmer , der für eine Vielzahl ganz unterschiedlicher pathogener Erreger eingesetzt werden kann (z. B. Bakterien, Viren, Parasiten, Protozoen oder Pilze). Dies ist ein entscheidender Vorteil gegenüber beispielsweise dem Einsatz von Antibiotika, die jeweils ganz spezifisch gegen den zu bekämpfenden Erreger gerichtet sein müssen. Durch die offenbarte Verwendung ist es möglich, gegen virale Infektionen vorzugehen, was bisher im allgemeinen in dieser Weise nicht möglich war. Zusätzlich wird durch die offenbarte Verwendung das Problem von sich entwickelnden Resistenzen der pathogenen Erreger umgangen, da der Wirkstoff nicht gegen den Erreger selbst, sondern gegen die von diesem Erreger in der Wirtszelle ausgelösten Reaktionen gerichtet ist.

Von der Erfindung werden neben den Hemmstoffen, die direkt die Aktivität der sauren Sphingomyelinase beeinflussen, auch Wirkstoffe umfaßt, die Vorstufen des Enzyms und/oder Aktivierungsmechanismen des Enzyms beeinflussen im Raum der Ansprüche. Weiterhin können die Wirkstoffe auch auf die Bildung, Stabilisierung, Mobilisierung und/oder Translokation der intrazellulären Vesikel wirken, in denen sich das Enzym in der Zelle befindet.

Zudem umfaßt die Erfindung die Verwendung von Hemmstoffen gemäss der Ansprüchen, die die biologische Wirkung der Produkte, die durch die enzymatische Reaktion der sauren Sphingomyelinase entstehen, beeinflussen. Bei diesen Produkten handelt es sich insbesondere um Ceramid, welches einen wesentlichen Bestandteil der Membranplattformen bildet. Von der Erfindung umfaßt sind daher Hemmstoffe gemäss der Ansprüchen die das Ceramid modifizieren und insbesondere inaktivieren, neutralisieren oder zerstören. Beispielsweise können erfindungsgemäß. Stoffe eingesetzt werden, die die Abbaurate von Ceramid erhöhen und unter die Ansprüche fallen. Hierbei ist insbesondere das Enzym Ceramid-Glukosyltransferase bevorzugt, das beispielsweise durch molekularbiologische Methoden in seiner Expression verstärkt und damit in seiner Aktivität verstärkt werden kann und/oder welches durch weitere Regulatoren aktiviert werden kann. Weiterhin können die Hemmstoffe gemäss der Ansprüchen die Zusammenlagerung der Ceramide verhindern, so daß die Plattformbildung beeinträchtigt wird. Zusätzlich umfaßt die Erfindung die Verwendung von Hemmstoffen gemäss der Ansprüchen, die die Funktionsfähigkeit der bereits gebildeten ceramidreichen Membranplattformen beeinflussen und/oder die Bildung der Membranplattformen von vornherein verhindern.

Vorteilhafterweise werden als Hemmstoffe pharmakologische Wirkstoffe eingesetzt, von denen bekannt ist, daß sie das Enzym saure Sphingomyelinase hemmen. Gemäss der Erfindung sind hierbei die trizyklischen un/oder tetrazyklischen Antidepressiva Amitryptilin und Imipramin verwendet Trizyklische Antidepressiva können beispielsweise einen proteolytischen Abbau des Enzyms bewirken, so daß das Enzym nicht mehr aktiv sein kann und eine Ceramidbildung verhindert wird. Durch diese Verminderung der Ceramidfreisetzung wird die Plattformbildung innerhalb der Zellmembran verhindert, so daß es nicht zu einer Infektion der Zelle kommt.

Hierbei handelt es sich um bekannte pharmakologische Wirkstoffe (Generika), die im wesentlichen keine Nebenwirkungen entfalten. Für die erfindungsgemäße Verwendung können diese Wirkstoffe in üblichen Darreichungsformen eingesetzt werden, insbesondere oral, intravenös, intramuskulär, topisch oder auch durch Inhalation. Weiterhin sind übliche Dosierungen für die erfindungsgemäße Verwendung geeignet. Allerdings kann der erfindungsgemäße Effekt auch mit reduzierten Dosierungen erreicht werden.

Neben den genannten Hemmstoffen kann auch Desipramin verwendet werden.

Bei der Prophylaxe und/oder Therapie von Krankheiten, deren Verlauf durch Infektionen beeinflusst wird nämlich zystischer Fibrose, kann es unter Umständen vorteilhaft sein, verschiedene der erfindungsgemäßen Wirkstoffe miteinander zu kombinieren.

Ein besonderer Vorteil der Erfindung ist, daß sich die Angriffspunkte der Wirkstoffe (Hemmstoffe), also insbesondere die saure Sphingomyelinase bzw. Ceramid, an der Zelloberfläche befinden. Diese Targets sind daher für die Wirkstoffe gut erreichbar, ohne daß es notwendig wäre, Die Erfindung umfaßt ferner eine pharmazeutische Zusammensetzung, zur Verwendung in der Prophylaxe und/oder Therapie von Zystischer Fibrose die mindestens einen Wirkstoff gemäß der erfindungsgemäßen Verwendung enthält, die trizyklischen und/oder tetrazyklischen Antidepressiva, Amitryptilin und/oder Imipramin.

Außerdem umfasst die Erfindung eine pharmazeutische Zusammensetzung zur Verwendung in der Prophylaxe und/oder Therapie von Zystischer Fibrose die mindestens eine wirksame Menge von Desipramin enthält. Als Darreichungsformen sind beispeilsweise Tabletten; Zäpfchen, Injektionslösungen oder Infusionslösungen geeignet.

Schließlich umfaßt die Erfindung die Behandlung von Krankheiten, deren Verlauf von Infektionen beeinflusst wird nämlich Zystischer Fibrose, wobei Hemmstoffe gemäss der Ansprüchen verabreicht werden, die das Enzym saure Sphingomyelinase und/oder Produkte der von diesem Enzym katalysierten Reaktion, insbesondere Ceramid, beeinflussen, vorzugsweise hemmen. Diese Behandlung erfolgt prophylaktisch und/oder während oder nach einer erfolgten Infektion. Eine vorbeugende Behandlung kann bei allgemeiner Infektionsgefahr oder bevorzugterweise bei einer akuten Infektionsgefahr durchgeführt werden. Bezüglich weiterer Merkmale dieser erfindungsgemäßen Behandlung wird auf die obige Beschreibung verwiesen.

Die genannten Merkmale und weitere Merkmale der Erfindung ergeben sich aus der nachfolgenden Beschreibung von Beispielen in Verbindung mit den Unteransprüchen und den Figuren. Hierbei können die einzelnen Merkmale jeweils für sich oder zu mehreren in Kombination miteinander verwirklicht sein. Die Beispiele und die Figuren gelten als Referenzen. In den Abbildungen zeigen:
- Fig. 1:: Die saure Sphingomyelinase (ASM) wird durch Infek- tion mit Rhinoviren aktiviert.
- Fig. 2:: Rhinoviren induzieren Ceramidfreisetzung.
- Fig. 3:: Amitryptilin und Imipramin hemmen dosisabhängig die Infektion menschlicher Zellen mit Rhinoviren.
- Fig. 4:: Die zytotoxische Wirkung von Rhinoviren wird durch Amitryptilin bzw. Imipramin gehemmt.
- Fig. 5:: HIV gp120 induziert die Freisetzung von Ceramid in humanen T-Lymphozyten.
- Fig. 6:: Zelluläre Stimulation mit gp120 induziert ceramidrei- che Membranplattformen.

### Beispiele

### 1. Infektion mit Rhinoviren

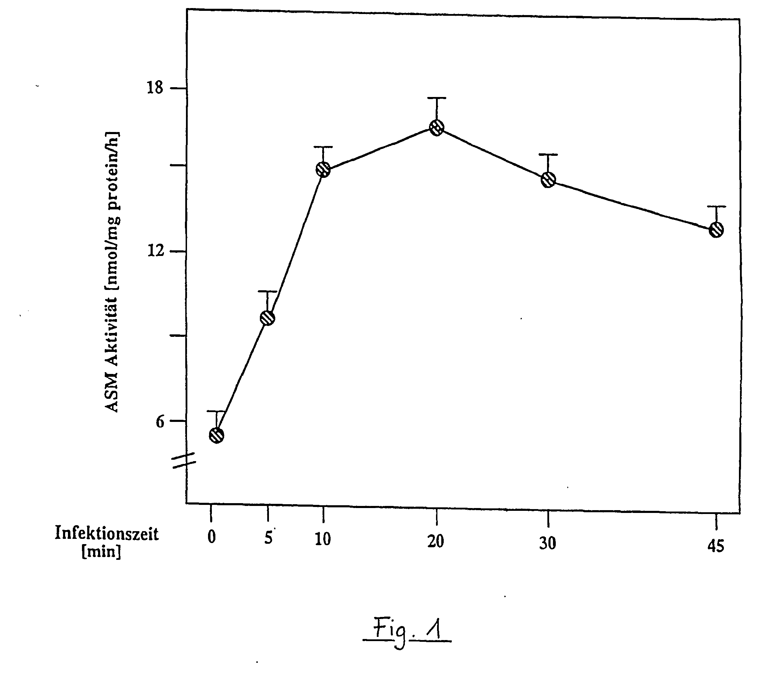

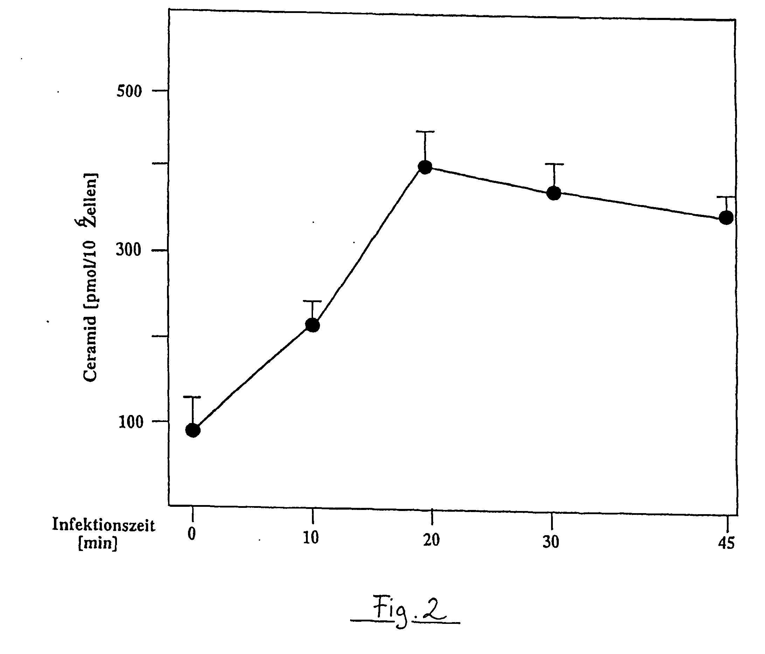

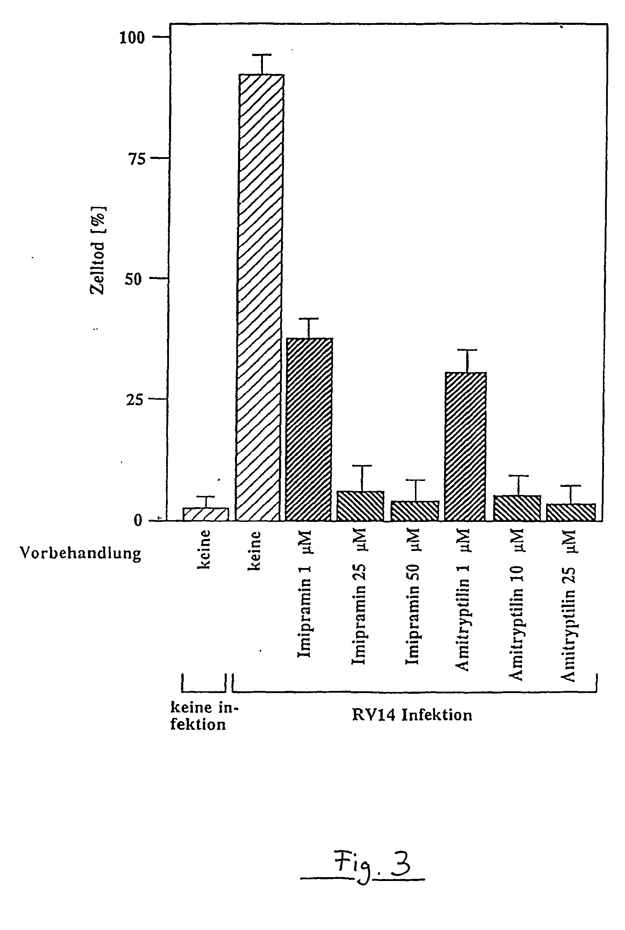

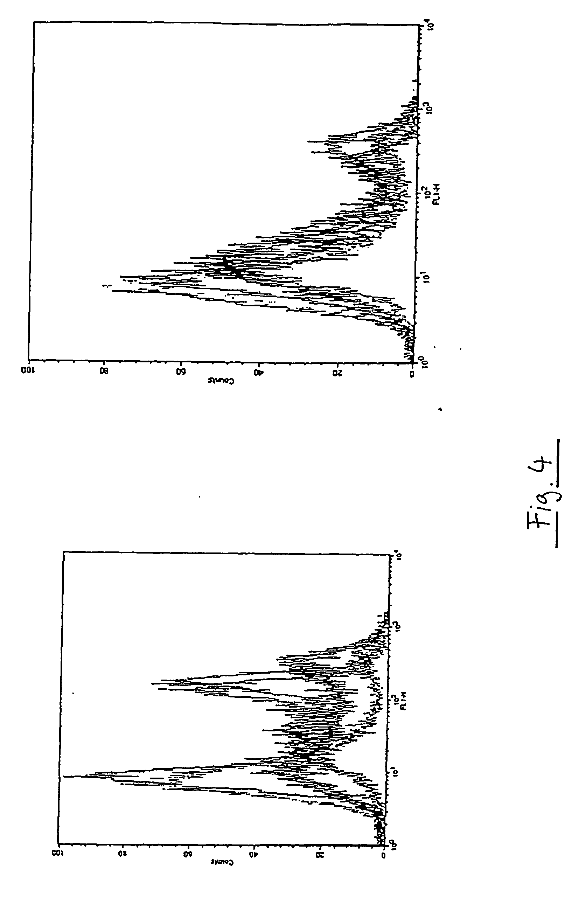

Die Ergebnisse der Versuche mit Rhinoviren zeigen, dass die Infektion humaner Epithelzellen mit verschiedenen Rhinoviren (HRV Stamm 14 und 16) zu einer Aktivierung der sauren Sphingomyelinase (Fig. 1) und zu einer Freisetzung von Ceramid (Fig. 2) führt.
a) Eine Infektion menschlicher Epithelzellen (Hela oder humane ex vivo Epithelzellen) aktiviert innerhalb von 10 bis 15 min. die saure Sphingomyelinase (ASM) um das Drei- bis Vierfache. Dies wurde am Beispiel von Hela-Zellen gezeigt. Die Ergebnisse sind in Fig. 1 zusammengefaßt. Die Zellen wurden mit Rhinoviren (Stamm 14, MOl von 25) infiziert und die Aktivität der Sphingomyelinase in Zellysaten gemessen. Dazu wurden die Zellen nach der Infektion gewaschen, in 250 mM Natriumacetat (pH 5,0), 1,3 mM EDTA und 0,05 % NP40 aufgenommen, durch Ultraschallbehandlung mit einem Stabsonicator bei niedriger Energie aufgebrochen und mit [¹⁴C]Sphingomyelin (0,5 µCi/Probe, 54,5 mCi/mmol; NEN) für 30 min. inkubiert. Der in vitro Enzymassay wurde durch Zugabe von 800 µl einer 2:1-Mischung aus CHCl₃ und CH₃OH (v/v) sowie 200 µl H₂O abgebrochen und die Freisetzung von [¹⁴C]Phosphorylcholin in den wäßrigen Überstand durch organische Extraktion szintigraphisch gemessen. Gezeigt sind die Mittelwerte ± Standardabweichung von drei Experimenten. Eine Vorinkubation der Zellen mit einem Hemmstoff der sauren Sphingomyelinase, i.e. Amitryptilin, blockiert die Aktivität der sauren Sphingomyelinase.
b) Die Stimulierung der sauren Sphingomyelinase korreliert mit einer Freisetzung von Ceramid aus den infizierten Zellen. Dies ist in Fig. 2 dargestellt. Eine Infektion humaner Epithelzellen (Hela) führt innerhalb weniger Minuten nach Infektion zu einer Freisetzung von Ceramid. Hierfür wurden wiederum Hela-Zellen mit Rhinoviren (Stamm 14) infiziert. Die Messung von Ceramid erfolgte durch einen Diacylglycerol (DAG)-Kinase Assay (Grassmé et al., Cell 91, 605-615, 1997). Ceramid wurde durch Zugabe von DAG-Kinase und [³²P]γATP zu [³²P]-Ceramid umgebaut. Das phosphorylierte Ceramid wurde dünnschichtchromatographisch aufgetrennt und szintigraphisch bestimmt. Dargestellt sind die Mittelwerte ± Standardabweichung von drei Experimenten. Eine Vorinkubation der Zellen mit Amitryptilin hebt die Freisetzung von Ceramid auf.
c) Die Infektion menschlicher Epithelzellen mit Rhinoviren induziert die Bildung ceramidreicher Membranplattformen. Sowohl die saure Sphingomyelinase als auch Ceramid sind auf der Oberfläche in Membranplattformen, an die auch die Rhinoviren binden, nach einer Infektion zu finden. Als experimenteller Nachweis hierfür wurden humane nasale Epithelzellen mit Rhinovirusstamm 14 über 20 min. infiziert, fixiert und mit Cy3-markierten monoklonalen Anti-Ceramid-Antikörpern (Alexis) gefärbt. Im Konfokalmikroskop zeigte sich kurze Zeit nach der Infektion die Bildung einer ceramidreichen Membranplattform. Nicht infizierte Zellen zeigten kein Ceramid auf der Zelloberfläche.
d) Eine pharmakologische Hemmung der sauren Sphingomyelinase blockiert die Infektion menschlicher Epithelzellen durch Rhinoviren dosisabhängig bis zu einer fast vollständigen Hemmung der Infektion. Als Pharmaka wurden die Pharmaka (Antidepressiva) Imipramin und Amitryptilin, die in Kontrollexperimenten innerhalb von 20 min. bis zu 98 % der Aktivität der sauren Sphingomyelinase blockieren, verwendet. Die Infektion menschlicher Epithelzellen durch Rhinoviren wurde in durchflußzytometrischen Analysen des zytopathischen Effektes der Viren gemessen. Da Rhinoviren in diesen Zellen Zelltod induzieren, kann der Zelltod als Maß für eine Infektion der Zellen verwendet werden. Die Dosiswirkungskurve der Hemmung der Infektion menschlicher Epithelzellen durch Imipramin und Amitryptilin ist in Fig. 3 dargestellt. Hierfür wurden humane Hela-Epithelzellen mit dem Rhinovirus-Stamm 14 für 24 h infiziert und die zytotoxische Wirkung der Viren durchflußzytometrisch nach Färbung mit FITC-Annexin gemessen. Amitryptilin bzw. Imipramin, die die saure Sphingomyelinase hemmen, wurden 30 min. vor Infektion der Rhinoviren in einem serumfreien Medium zu den Zellen gegeben. Die Daten zeigen, daß die Pharmaka die virale Infektion fast vollständig hemmen. Amitryptilin und Imipramin hatten selbst keine zytotoxische Wirkung auf die Zellen. Gezeigt sind die Mittelwerte ± Standardabweichung von drei Experimenten.
e) Die zytotoxische Wirkung von Rhinoviren wird durch Amitryptilin bzw. Imipramin gehemmt. Fig. 4 zeigt die repräsentative durchflußzytometrische Analyse der Hemmung der Infektion von Hela-Zellen durch Rhinoviren nach Behandlung mit Amitryptilin. Hierfür wurden humane Hela-Epithelzellen mit verschiedenen Rhinovirus-Stämmen (RV14, RV16) 24 h infiziert. Die Infektion wurde anhand der zytotoxischen Wirkung der Viren gemessen und durch Färbung der Zellen mit FITC-Annexin in einem Durchflußzytometer bestimmt. Eine Rechtsverschiebung der Kurve bedeutet einen Anstieg der FITC-Annexinbindung und ist damit ein Maß für Zelltod. Amitryptilin bzw. Imipramin als Hemmstoffe der sauren Sphingomyelinase wurden 30 min. vor Infektion mit den Rhinoviren zu den Zellen in einem serumfreien Medium zugegeben. Die Daten zeigen, daß die Pharmaka die virale Infektion fast vollständig hemmen. Gezeigt ist die Wirkung von Amitryptilin, analoge Daten wurden für Imipramin erhoben. Die linke Darstellung in Fig. 4 zeigt den Effekt ohne Wirkstoffzugabe, die rechte Darstellung den Effekt mit Wirkstoffzugabe.
f) Durch eine Behandlung von Hela-Zellen mit anti-Ceramid-Antikörpern wird eine Infektion der Zellen mit Rhinoviren nahezu vollständig gehemmt. Für entsprechende Versuche wurden anti-Ceramid-Antikörper (Fa. Alexis) in einer Konzentration von 5 ng/ml mit den Viren (Rhinovirus-Stämme 2, 14 und 16) zu den Zellen gegeben. Die Infektion der Zellen mit den Rhinoviren wurde um etwa 95% gehemmt.

### 2. Infektion mit HIV

HIV infiziert humane Zellen im wesentlichen durch die Bindung des gp120 Moleküles des Virus an den CD4-Rezeptor. Ohne die Bindung von gp120 an CD4 kommt es nur zu einer ineffizienten Infektion von Zellen mit HIV. Das gp120 Molekül bildet mit dem gp41 Molekül einen oligomeren Komplex, in dem gp120 als Trimer vorliegt. Die Bindung von gp120 an das CD4=Molekül auf T-Lymphozyten verändert die Konformation von gp120, insbesondere kommt es zu einer Veränderung der Konformation des variablen Loops, wodurch die sog. Co-Rezeptor-Bindungsstelle freigelegt wird. Über diese Bindungsstelle bindet gp120 an einen Co-Rezeptor, meist die Zytokinrezeptoren CCR5 oder CXCR4. Insgesamt wurden mehr als 14 verschiedene Co-Rezeptoren identifiziert, von grosser in vivo Bedeutung scheinen aber nur CCR5 oder CXCR4 zu sein. Durch die Bindung von HIV an CD4 und weitere Co-Rezeptoren wird die Aufnahme des Virus in die Zelle initiiert (Clapham P.R., McKnight A. (2001). HIV-1 receptors and cell tropism. British Medical Bulletin 58: 43-59).

Das CD4-Molekül befindet sich bereits konstitutiv, d.h. auch in nicht infizierten Zellen, in Rafts. Nach der Infektion mit HIV kommt es zu einer Umverteilung von CD4 und zu einer Konzentration von CD4 in einem relativ kleinen Bereich der Zellmembran (Popik W., Alce T.M., Au W.C. (2002). Human lmmunodeficiency Virus Type 1 uses lipid raftcolocalized CD4 and chemokine receptors for productive entry into CD4+T cells. J. of Virology 76: 4709-4722). Dieses Phänomen einer lokalen, sehr hohen Anreicherung eines Moleküles bezeichnet man als Clustering bzw. Aggregation. CD4 ko-lokalisiert nach Infektion in diesen Clustern mit GM1, einem typische Marker für Rafts, und auch CCR5 und CXCR4, die nach Stimulation in die neu gebildeten Membranplattformen rekrutiert werden. Werden Rafts durch Vorinkubation der Zellen mit Reagenzien, die Colesterol extrahieren, zerstört, wurde in diesen Studien sowohl das Aggregieren von CD4 nach Infektion als auch die Infektion der T-Zellen selbst verhindert. Die Bindung des Virus an CD4 war dagegen nicht verändert. Dies zeigt bereits, dass Membranrafts eine herausragende Rolle bei der Infektion humaner Zellen mit HIV spielen. Die Mechanismen, die die Fusion vieler kleiner Rafts zu grossen Membranplattformen, die dem Aggregieren von CD4 sowie der Rekrutierung von Co-Rezeptoren dienen, und letztlich die Infektion zu vermitteln scheinen, waren jedoch bislang unbekannt.

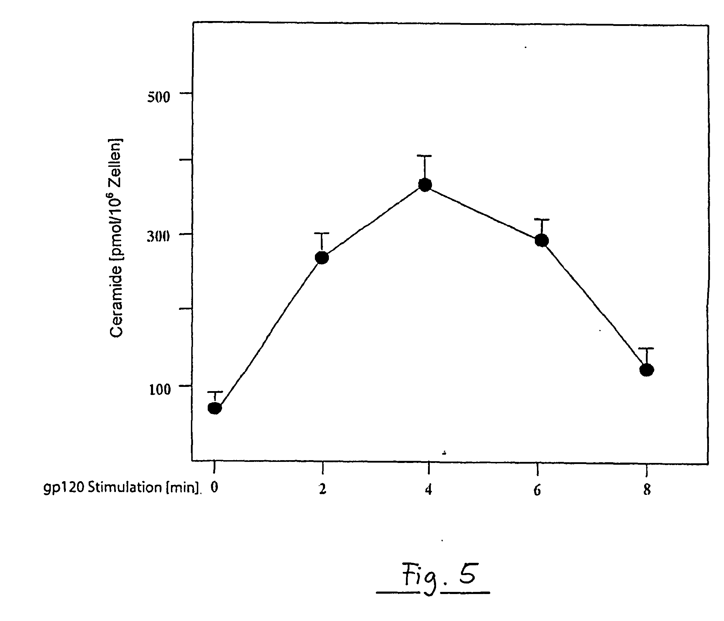

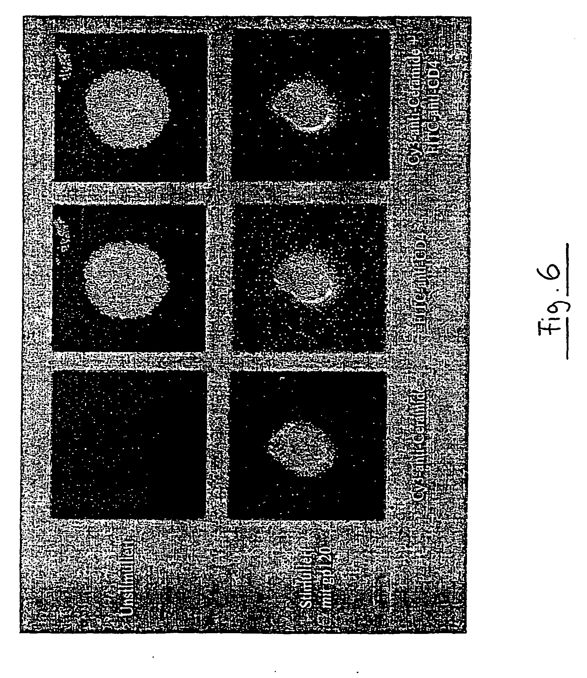

Die folgenden Ergebnisse zeigen anhand einer Stimulation humaner T-Lymphozyten mit rekombinantem gp120, das HIV ceramidreiche Membranplattformen benutzt, um humane Zellen zu infizieren.
a) Innerhalb weniger Minuten induziert gp 120 die Freisetzung von Ceramid in humanen T-Lymphozyten. Fig. 5 zeigt die Behandlung humaner CD4-positiver Lymphozyten mit 10 µg/ml rekombinantem gp120. Innerhalb von 1 Minute wird Ceramid freigesetzt. Ceramid wurde mit einem DAG-Kinase Assay bestimmt.
b) Die Freisetzung von Ceramid aus humanen T-Lymphozyten nach Stimulation mit gp120 korreliert mit der Bildung ceramidreicher Membranplattformen in der Zellmembran stimulierter Zellen, in denen das CD4-Molekül ko-lokalisiert und Cluster bildet. Fig. 6 zeigt, dass die zelluläre Stimulation mit gp 120 ceramidreiche Membranplattformen induziert. Die Stimulation humaner CD4-positiver T-Zellen mit gp120 (10 µg/ml) führt innerhalb von 2 Minuten zur Bildung ceramidreicher Membranplattformen. Die Bildung ceramidreicher Membrandomänen wurde nach Markierung der Zellen mit einem Cy3-markierten anti-Ceramid-Antikörper mittels Fluoreszenzmikroskopie bestimmt. In diesen ceramidreichen Membranplattformen aggregiert CD4, das mit einem FITC-markierten Antikörper dargestellt wurde. Quantitative Analysen der Bildung ceramidreicher Membranplattformen zeigen, dass 50 ± 7% aller CD4-positiven T-Lymphozyten 5 Min nach Stimulation mit gp120 eine ceramidreiche Membranplattform aufweisen.

### 3. Infektion mit Pseudomonas aeruginosa

Die Bedeutung ceramidreicher Membranplattformen für Infektionen mit pathogenen Bakterien und Viren wurde am Beispiel der Infektion mammalischer Zellen mit *Pseudomonas aeruginosa* gezeigt. Die Aktivierung der sauren Sphingomyelinase und die Freisetzung von Ceramid wurden sowohl in vitro nach Infektion von Chang-Epithelzellen, WI-38 Fibroblasten, ex-vivo Lungenfibroblasten, ex-vivo kultivierten Tracheal-Epithelzellen als auch in vivo in Epithelzellen der Trachea nach Infektion mit *P. aeruginosa* beobachtet. Die Freisetzung von Ceramid nach Infektion erfolgt in Rafts, die durch das freigesetzte Ceramid zu grossen Membranplattformen umorganisiert werden. Das freigesetzte Ceramid und die saure Sphingomyelinase lokalisieren in den neu gebildeten Membranplattformen nach Infektion mit *P*. *aeruginosa.*

Die Bedeutung der sauren Sphingomyelinase für die Bildung von Membranplattformen nach *P*. *aeruginosa* Infektion zeigt sich an dem völligen Fehlen ceramidreicher Membranplattformen nach Infektion von saure Sphingomyelinase-defizienten Zellen. Die Rolle ceramidreicher Membranplattformen für die Infektion mit *P*. *aeruginosa* wurde in saure Sphingomyelinase-defizienten Zellen bzw. durch Zerstörung von Rafts mit Pharmaka, die mit dem Cholesterol-Stoffwechsel interferieren, untersucht. Bei diesen Pharmaka handelt es sich um β-Cyclodextrin, Nystatin und Filipin. Der Entzug von Cholesterol aus Membranrafts führt zu einem Kollaps von Rafts. In vivo wurden Rafts in der Lunge durch pulmonale Lavage mit β-Cyclodextrin, Nystatin und Filipin zerstört bzw. normale und saure Sphingomyelinase-defiziente Mäuse verwendet. Die Ergebnisse zeigen, dass ceramidreiche Membranplattformen die Internalisierung der Bakterien in Epithelzellen, den Tod infizierter Zellen und die Freisetzung pro-inflammatorischer Zytokine regulieren.

Die in vivo Bedeutung dieser Befunde wurde in Infektionsversuchen mit normalen und saure Sphingomyelinase-defizienten Mäusen gezeigt. Während normale Mäuse eine pulmonale Infektion mit *P. aeruginosa* innerhalb weniger Tage ausheilen, waren die saure Sphingomyelinase-defizienten Mäuse sehr empfindlich gegenüber einer pulmonalen *P. aeruginosa* Infektion und verstarben innerhalb weniger Tage nach Infektionsbeginn an einer Sepsis.

## Patentansprüche

1. Verwendung von Hemmstoffen der sauren Sphingomyelinase und/ oder von Hemmstoffen von Produkten der durch dieses Enzym katalysierten Reaktion, insbesondere von Ceramid, zur Herstellung einer pharmazeutischen Zusammensetzung zur Prophylaxe und/oder Therapie von zystischer Fibrose, **dadurch gekennzeichnet, dass** die Hemmstoffe Antidepressiva sind, wobei die Antidepressiva Amitryptilin, Imipramin und/oder Desipramin sind.

2. Amitryptilin, Imipramin und/oder Desipramin zur Verwendung in der Prophylaxe und/oder Therapie von zystischer Fibrose.

## Claims

1. Use of inhibitors of acid sphingomyelinase and/or inhibitors of products of the reaction catalysed by said enzyme, in particular of ceramide, for the production of a pharmaceutical composition for prophylaxis and/or treatment of cystic fibrosis, **characterised in that** the inhibitors are anti-depressants, the anti-depressants being amitryptiline, imipramine and/or desipramine.

2. Amitryptiline, imipramine and/or desipramine for use in prophylaxis and/or treatment of cystic fibrosis.

## Revendications

1. Utilisation d'inhibiteurs de la sphingomyélinase acide et/ou d'inhibiteurs de produits de la réaction catalysée par cette enzyme, en particulier de céramide, pour la fabrication d'une composition pharmaceutique destinée à la prophylaxie et/ou à la thérapie de la fibrose kystique, **caractérisée en ce que** les inhibiteurs sont des antidépresseurs, les antidépresseurs étant l'amitryptiline, l'imipramine et/ou la désipramine.

2. Amitryptiline, imipramine et/ou désipramine pour une utilisation dans la prophylaxie et/ou la thérapie de la fibrose kystique.
